(19) Europäisches Patentamt
European Patent Office
Office européen des brevets

(11) **EP 2 215 966 A2**

(12) **EUROPEAN PATENT APPLICATION**

(43) Date of publication:
**11.08.2010 Bulletin 2010/32**

(51) Int Cl.:
**A61B 5/08** (2006.01)

(21) Application number: **10150576.6**

(22) Date of filing: **12.01.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **10.02.2009 JP 2009029039**

(71) Applicant: **TANITA CORPORATION**
**Tokyo 174-8630 (JP)**

(72) Inventor: **Masuo, Yoshihisa**
**Itabashi-ku Tokyo 174-8630 (JP)**

(74) Representative: **Haley, Stephen**
**Gill Jennings & Every LLP**
**Broadgate House**
**7 Eldon Street**
**London EC2M 7LH (GB)**

(54) **Respiration Type Evaluation Apparatus**

(57) A respiration type evaluation apparatus (1) includes a bioelectrical impedance determiner (170,200) for determining a bioelectrical impedance at at least a body region of a human subject that contributes to respiration of the human subject, and a respiration type determiner (170) for determining whether respiration of the human subject is abdominal or costal on the basis of change over time of the bioelectrical impedance determined by the bioelectrical impedance determiner.

Fig. 1

**EP 2 215 966 A2**

## Description

The present invention relates to respiration type evaluation apparatuses for determining the type of respiration of human subjects.

There have been know various apparatuses for determining bioelectrical impedance and for estimating body conditions based on the determined impedance. For example, JP-A-2007-50127 discloses a technique for estimating the capacity of the lungs of a human subject on the basis of the impedance of the body trunk.

Respiration (breathing) is distinctive among vital actions (e.g., blood pressure, body temperature, skin temperature, brain waves, and pulse waves) because it can be under voluntary control. Accordingly, many methods have been developed around the world for maintaining health, e.g., *yoga, Qigong,* chiropractic, *zazen,* or weight control. Respiration is classified into abdominal (diaphragmatic breathing) and costal (chest breathing). Abdominal respiration is linked with not only various methods of health maintenance, but also voice training.

It may be sometimes advantageous to determine exactly whether respiration of a human subject is abdominal or costal. However, there has not been a technique for determining whether respiration of a human subject is abdominal or costal on the basis of bioelectrical impedance data of the human subject.

Accordingly, the present invention provides respiration type evaluation apparatuses for determining whether respiration of a human subject is abdominal or costal on the basis of bioelectrical impedance of the human subject.

According to the present invention, there is provided a respiration type evaluation apparatus including: a bioelectrical impedance determiner for determining a bioelectrical impedance at at least a body region of a human subject that contributes to respiration of the human subject; and a respiration type determiner for determining whether respiration of the human subject is abdominal or costal on the basis of change over time of the bioelectrical impedance determined by the bioelectrical impedance determiner.

When a human performs respiratory actions that consist of inhalation and exhalation, bioelectrical impedances at muscles and viscera of the human contributing to respiration change. The aspect of change of the bioelectrical impedances in abdominal respiration may be different from that in costal respiration. Accordingly, on the basis of change over time of the bioelectrical impedance at at least a body region of a human subject that contributes to respiration, it is possible to determine whether respiration of the human subject is abdominal or costal.

Different body regions have different compositions, so that different body regions move differently from each other in costal respiration and abdominal respiration, and aspects of change of bioelectrical impedances are different depending on the body region and on the type of respiration. More specifically, the aspect of impedance change in each exhalation is remarkably different depending on the body region and on the type of respiration. Preferably, the bioelectrical impedance determiner may determine bioelectrical impedances at plural body regions, and the respiration type determiner may analyze change over time of each bioelectrical impedance at plural body regions, thereby determining whether respiration of the human subject is abdominal or costal.

The change over time of the bioelectrical impedance at the body region includes periodical change of the bioelectrical impedance influenced by respirations of the human subject, each including an inhalation and an exhalation. Preferably, the respiration type determiner determines whether respiration of the human subject is abdominal or costal on the basis of a difference between change over time of the bioelectrical impedance in an inhalation of the human subject and change over time of the bioelectrical impedance in an exhalation of the human subject.

The aspect of impedance change in each exhalation is remarkably different depending on the type of respiration, i.e., costal respiration and abdominal respiration. This feature is particularly prominent in the impedance at the abdominal region. Accordingly, by analyzing the difference between change over time of the bioelectrical impedance in an inhalation at the abdominal region of the human subject and change over time of the bioelectrical impedance in an exhalation at the abdominal region of the human subject, it is possible to determine whether respiration of the human subject is abdominal or costal. Preferably, the respiration type determiner determines whether respiration of the human subject is abdominal or costal on the basis of a difference between change over time of the bioelectrical impedance in an inhalation at the abdominal region of the human subject and change over time of the bioelectrical impedance in an exhalation at the abdominal region of the human subject.

The bioelectrical impedance determiner may determine a first bioelectrical impedance at a first region of the human subject including the lungs and a second bioelectrical impedance at a second region of the human subject, the second region being specified such that the second bioelectrical impedance changes in an opposite manner to the change of the first bioelectrical impedance when the human subject performs exhalation in abdominal respiration. The respiration type determiner may determine whether respiration of the human subject is abdominal or costal on the basis of a difference between change over time of the first bioelectrical impedance and change over time of the second bioelectrical impedance.

Lungs of human subjects expand and contract in a similar way in costal respiration and abdominal respiration. The region at which bioelectrical impedance varies most greatly due to respiration among regions that contributes to respiration is the lungs. The bioelectrical impedance at the costal respiratory muscle around the lungs changes in a manner similar to that at the lungs. Accordingly, change over time of the first bioelectrical impedance is not significantly different depending on costal respiration and abdominal respiration, but significantly reflects respiration actions including inhalation and exhalation. On the other hand, the second region is specified such that the second bioelectrical impedance changes in an opposite manner to the change of the first bioelectrical impedance when the human subject performs an exhalation in abdominal respiration. Thus, in abdominal respiration, the aspect of change over time of the first bioelectrical impedance is different from the aspect of change over time of the second bioelectrical impedance. Accordingly, by analyzing the difference between change over time of the first bioelectrical impedance and change over time of the second bioelectrical impedance, it is possible to determine whether respiration of the human subject is abdominal or costal. In the analysis of the difference, degrees of abdominal respiration and costal respiration may be determined for respiration of the human subject on the basis of the extent of the difference.

More specifically, the bioelectrical impedance determiner may determine the first bioelectrical impedance at the first region including the lungs of the human subject and excluding the abdomen of the human subject and may determine the second bioelectrical impedance at the second region including the abdomen of the human subject. The abdomen includes the diaphragm, the visceral tissue, and the abdominal skeletal muscle. In exhalations of abdominal respiration, the visceral tissue expands in the perpendicular direction so as to move up the diaphragm due to ventrodorsal contraction of the abdominal skeletal muscle, and therefore the second bioelectrical impedance increases in exhalations of abdominal respiration. This is not the case with costal respiration. On the other hand, in both abdominal respiration and costal respiration, the diaphragm moves up to compress the lungs at exhalations and moves down to expand the lungs at inhalations, so that the first bioelectrical impedance changes in a similar manner in both of abdominal respiration and costal respiration. Accordingly, by analyzing the difference between change over time of the first bioelectrical impedance and change over time of the second bioelectrical impedance, it is possible to determine whether respiration of the human subject is abdominal or costal.

The first region may be the upper body trunk of the human subject and the second region may be the middle body trunk. As will be described below, the difference between bioelectrical impedance at the upper body trunk and bioelectrical impedance at the middle body trunk corresponds to the bioelectrical impedance at the abdominal region.

Preferably, the limb-lead eight-electrode method is used in which electrodes are arranged at both hands and both soles. It can be contemplated that in order to determine the bioelectrical impedance at the abdominal region, electrodes may be arranged directly at the abdominal region, for example, at the epigastric fossa and subumbilically, but arranging the electrodes at the abdominal region is troublesome and may be undesirable to the human subject. According to the limb-lead eight-electrode method, by arranging electrodes at both hands and both soles, bioelectrical impedance at the upper body trunk and upper extremities and bioelectrical impedance at the middle body trunk can be determined without arranging electrodes at the body trunk. By arranging electrodes at both hands, the impedance determined for the upper body trunk reflects the impedance at the lungs, but is affected by impedance at right and left upper extremities. However, if right and left upper extremities do not move, the bioelectrical impedances thereat do not change and do not affect determination of costal respiration and abdominal respiration. Alternatively, if the bioelectrical impedances at right and left upper extremities are determined, the impedance determined for the upper body trunk can be compensated to exclude the influence of the upper extremities.

In the respiration type evaluation apparatus, the respiration type determiner may include a difference calculator for calculating an impedance difference between the first bioelectrical impedance and the second bioelectrical impedance, and the respiration type determiner may determine whether respiration of the human subject is abdominal or costal on the basis of change over time of the impedance difference. The expression, "determine ... on the basis of change over time of the impedance difference" may include an analysis with the use of pattern matching techniques. For example, conformity between measured change of the impedance difference and prepared change of the bioelectrical difference for abdominal respiration is calculated, whereas conformity between measured change of the bioelectrical difference and prepared change of the bioelectrical difference for costal respiration is also calculated. On the basis of the calculation results, it is possible to determine whether respiration of the human subject is abdominal or costal.

The difference calculator may include a compensator for compensating at least one of the first bioelectrical impedance or the second bioelectrical impedance, and the difference calculator may calculate the impedance difference between the first bioelectrical impedance and the second bioelectrical impedance among which at least one is compensated by the compensator. Preferably, the compensator compensates at least one of the bioelectrical impedances, so as to enlarge the impedance difference therebetween.

The compensation may include, for example, normalization of change of the determined bioelectrical impedance to the average impedance or the median impedance. In addition, or instead, determined bioelectrical impedance may be compensated on the basis of personal or individual body indexes or parameters, such as the age, height, weight, or body fat ratio of the human subject. In this case, the compensated bioelectrical impedances may exclude effects due to variation of body composition, for example, of the visceral tissue, the abdominal skeletal muscle, visceral fat mass, or subcutaneous fat mass, thereby enhancing reliability of the determined type of respiration or the degree or depth of respiration.

The compensator may compensate the bioelectrical impedance so that change over time of the impedance difference in inhalations conform with a statistical change over time of the impedance difference, and the respiration type determiner may determine whether respiration of the human subject is abdominal or costal on the basis of change over time of the impedance difference in exhalations. More specifically, the compensator may multiply the determined bioelectrical impedance by a compensation factor to produce a compensated bioelectrical impedance. The compensation factor may be set in such a manner that change of the compensated bioelectrical impedance in inhalations conform with statistical change of bioelectrical impedance in inhalations.

The respiration type determiner may include a comparer for comparing the impedance difference with a threshold, and the respiration type determiner may determine whether respiration of the human subject is abdominal or costal on the basis of the comparison at the comparer.

The respiration type determiner may include an integrator for deriving an integration of the impedance difference, and the respiration type determiner may determine whether respiration of the human subject is abdominal or costal on the basis of the integration of the impedance difference derived at the integrator.

The respiration type evaluation apparatus may further include a respiration period decider for deciding a respiration period in which an inhalation and an exhalation are performed on the basis of a cycle of variation of the first bioelectrical impedance determined by the bioelectrical impedance determiner. Since the first bioelectrical impedance relates to the first region including the lungs and reflects movement of the lungs, the respiration period decider can determine an accurate respiration period. If a respiration rhythm guidance device is provided for guiding the human subject about a target respiration pattern or rhythm by sound or vision, the respiration period can be decided more accurately.

The integrator may integrate the impedance difference over a threshold to produce a first integral of the impedance difference in an inhalation for each respiration period, and may integrate the impedance difference beneath the threshold to produce a second integral of the impedance difference in an exhalation for each respiration period. The respiration type determiner may include an integral difference calculator for calculating an integral difference between the first integral and the second integral for each respiration period. The respiration type determiner may determine whether respiration of the human subject is abdominal or costal on the basis of the integral difference calculated by the integral difference calculator. The second bioelectrical impedance changes in an opposite manner to change of the first bioelectrical impedance when the human subject performs exhalation in abdominal respiration. On the other hand, change over time of the first bioelectrical impedance is not significantly different depending on costal respiration and abdominal respiration. Therefore, on the basis of the integral difference, degrees of the abdominal respiration and costal respiration can be determined.

The respiration type determiner may determine a degree of abdominal respiration and a degree of costal respiration with reference to the respiration of the human subject. The respiration type evaluation apparatus may further include a first reporter for reporting the degrees determined by the respiration type determiner. In this case, the degrees determined by the respiration type determiner can be utilized as useful biofeedback information for respiration training.

The respiration type evaluation apparatus may further include a second reporter for instructing the human subject of a time and a depth of inhalation that the human subject should perform and a time and a depth of exhalation that the human subject should perform. This may facilitate pattern matching of respiration period including an inhalation and an exhalation. It is possible to guide the human subject about a target respiration pattern or rhythm.

The respiration type evaluation apparatus may further include a display device for showing change of depth of each exhalation and inhalation on the basis of the bioelectrical impedance of the at least one body region determined by the bioelectrical impedance determiner. The shown change of depth can be utilized as useful biofeedback information on the respiration pattern.

BRIEF DESCRIPTION OF THE DRAWINGS

With reference to the accompanying drawings, various embodiments of the present invention will be described hereinafter. In the drawings:

Fig. 1 is a block diagram showing an electrical structure of a body condition determination apparatus 1 of an embodiment according to the present invention;
Fig. 2 is a perspective view showing the body condition determination apparatus;
Fig. 3 is an enlarged view of a part of Fig. 2;
Fig. 4 is a diagram for explaining body regions determined by using selected current electrodes and selected voltage electrodes;
Fig. 5 is a flow chart showing an operation of the body condition determination apparatus;
Fig. 6 is a schematic view showing tissues in the body trunk of humans;
Fig. 7A shows an equivalent circuit model of the middle body trunk of humans;
Fig. 7B shows an equivalent circuit model of the upper body trunk of humans;

Fig. 8 is a diagram showing relationships between respiration and change in bioelectrical impedance;

Fig. 9 is a flow chart showing an example of a respiration analysis process executed in the body condition determination apparatus;

Fig. 10 is a graph showing change over time of each of bioelectrical impedances at the middle body trunk and the upper body trunk;

Fig. 11 is a graph showing change over time of the difference between bioelectrical impedances at the middle body trunk and the upper body trunk;

Fig. 12 is a waveform diagram showing change over time of a comparison result signal produced by the body condition determination apparatus;

Fig. 13 is a flow chart showing an example of a respiration type determination process executed in the body condition determination apparatus;

Fig. 14 is a flow chart showing another example of a respiration analysis process executed in the body condition determination apparatus;

Fig. 15 is a view showing an image shown on a display device of the body condition determination apparatus;

Fig. 16 is a view showing another image shown on the display device;

Fig. 17 is a view showing another image shown on the display device;

Fig. 18 is a view showing another image shown on the display device;

Fig. 19 is a view showing another image shown on the display device; and

Fig. 20 is a view showing another image shown on the display device.

## DETAILED DESCRIPTION OF PREFERRED EMBODIMENTS STRUCTURE

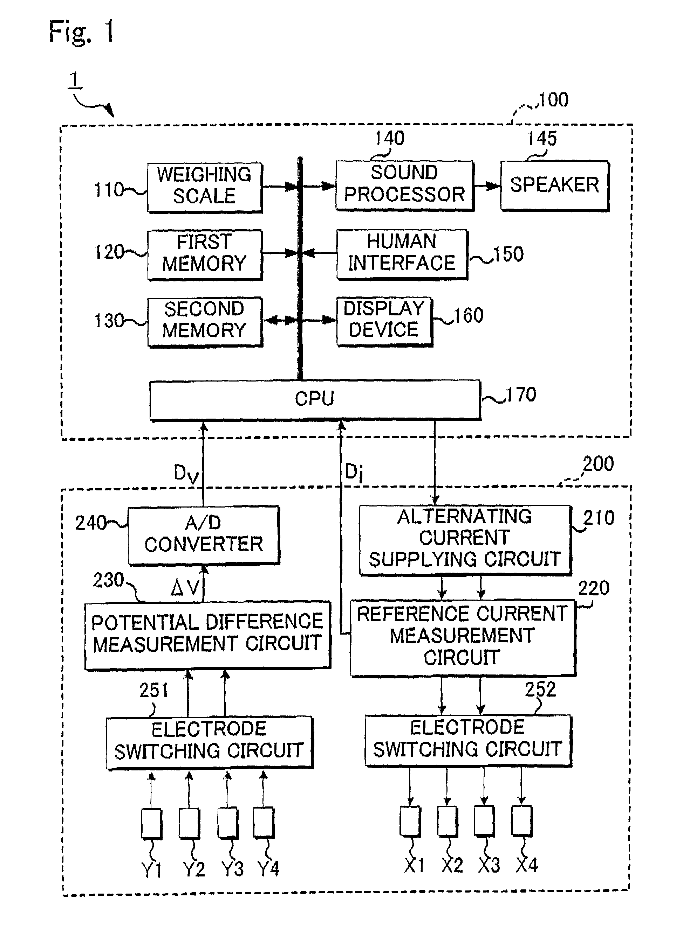

A body condition determination apparatus 1 shown in Fig. 1 determines conditions of human subjects, and functions as a respiration type evaluation apparatus that determines whether respiration of the human subject is abdominal or costal, and determines the degree of abdominal respiration and the degree of costal respiration.

The body condition determination apparatus 1 includes a management part 100 for measuring the body weights of human subjects and for managing overall operations of the body condition determination apparatus 1, and a bioelectrical impedance determination part 200 for determining bioelectrical impedances at various body regions of human subjects.

The management part 100 includes a weighing scale 110, a first memory 120, a second memory 130, a sound processor 140, a speaker 145, a human interface 150, and a display device 160. These elements are connected with a processor that is typically a CPU (Central Processing Unit) 170 via a bus. The CPU 170 serves as a main controller for controlling the entire apparatus. During operation of the CPU 170, the CPU 170 receives clock signals from a clock signal generation circuit (not shown). When a power switch (not shown) is turned on, a power source circuit supplies these elements with power.

The weighing scale 110 measures weights of human subjects and supplies weight data to the CPU 170 via the bus.

The first memory 120 is a nonvolatile memory, for example, a ROM (Read Only Memory). The first memory 120 stores a control program for controlling the entire apparatus. In accordance with the control program the CPU 170 executes a predetermined arithmetic process, thereby determining whether respiration of the human subject is abdominal or costal.

The second memory 130 is a volatile memory, for example, a DRAM (Dynamic Random Access Memory). The second memory 130 serves as a work area for the CPU 170. During the execution of the predetermined arithmetic process by the CPU 170, the second memory 130 stores various data.

Under control by the CPU 170, the sound processor 140 conducts digital-to-analog conversion on sound data, amplifies the resulting sound signals to the speaker 145, and supplies the amplified sound signals to the speaker 145. The speaker 145 converts the amplified sound signals into sound vibrations and emits sound. Accordingly, the speaker 145 can provide human subjects with advisory or informative sounds, for example, guidance in respiration rhythm.

The human interface 150 includes input devices. The human subject manipulates the input devices in order to input personal information on the human subject, for example, the height, age, and sex into the body condition determination apparatus 1.

The display device 160 shows the measurement results, such as the weight or the type of respiration. The display device 160 also shows instructions to exhale and inhale in order to lead the human subject to perform abdominal respiration. The display device 160 shows messages for leading the human subject to input various information into the human interface 150. The display device 160 may be, for example, a liquid crystal display device.

The bioelectrical impedance determination part 200 is used for determining bioelectrical impedance. The bioelectrical impedance determination part 200 includes an alternating current supplying circuit 210, a reference current measurement circuit 220, a potential difference measurement circuit 230, an A/D converter 240, and electrode switching circuits 251 and 252.

The alternating current supplying circuit 210 generates a reference current $I_{ref}$ having a frequency determined in the control program. The reference current measurement circuit 220 supplies the reference current $I_{ref}$ to the human subject, measures the actual value of the reference current $I_{ret}$ flowing through the human subject, and supplies electric current data $D_i$ indicative of the actual value of the reference current $I_{ref}$. The electrode switching circuit 252 selects, among four alternating current electrodes X1 through X4, two or four electrodes through which the current flows. The current electrodes should be brought into contact with the human subject.

The potential difference measurement circuit 230 measures the potential difference between two voltage electrodes selected from among four voltage electrodes Y1 through Y4 by the other electrode switching circuit 251, and generates a potential difference signal $\Delta V$ indicative of the potential difference. The A/D converter 240 converts the analog potential difference signal $\Delta V$ into a digital signal, namely, voltage data $D_v$, and supplies the voltage data $D_v$ to the CPU 170. The CPU 170 calculates the bioelectrical impedance Z on the basis of the voltage data $D_v$ and the current data $D_i$. That is, the bioelectrical impedance Z is the potential difference divided by the current. Thus, the CPU 170 and the bioelectrical impedance determination part 200 serve as a bioelectrical impedance determiner for determining bioelectrical impedance at at least a body region of the human subject.

The first memory 120 may store various data in advance. For example, the first memory 120 may store correlation equations or tables for deriving the body fat percentage and the amount of muscle mass on the basis of bioelectrical impedances at various body regions.

The CPU 170 calculates the weight and bioelectrical impedances at various body regions (e,g., bioelectrical impedances at the upper limbs, bioelectrical impedances at the lower limbs, and the bioelectrical impedance at the body trunk), and controls various operations including signal inputting, signal outputting, measurements, calculations. The CPU 170 can calculate the ratio of visceral fat to subcutaneous fat, the visceral fat amount, the subcutaneous fat ratio, the subcutaneous fat amount, the systemic body fat ratio, and body regional fat ratios (e.g., body fat ratios at the upper limbs, the lower limbs, and the body trunk).

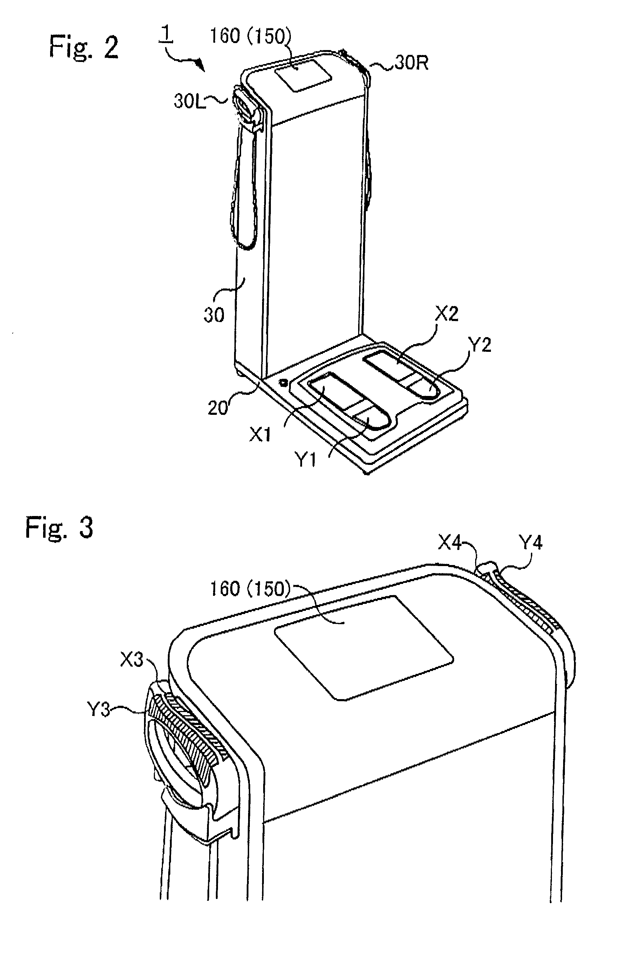

Fig. 2 shows the appearance of the body condition determination apparatus 1. The body condition determination apparatus 1 has an L-shape including a platform 20 on which the human subject stands and a rectangular-columnar housing 30 extending upwardly from the platform 20. The platform 20 is provided with a left-foot current electrode X1 and a left-foot voltage electrode Y1 on which the left foot of the human subject will be placed, and a right-foot current electrode X2 and a right-foot voltage electrode Y2 on which the right foot of the human subject will be placed.

At the top of the housing 30, the aforementioned display device 160 is located. The display device 160 is a touch panel and serves as the human interface 150.

A left electrode handle 30L is located at the left side surface of the housing 30 whereas a right electrode handle 30R is located at the right side surface of the housing 30.

Fig. 3 is an enlarged view showing the upper part of the housing 30. As shown in Fig. 3, the left electrode handle 30L includes a left-hand current electrode X3 and a left-hand voltage electrode Y3 whereas the left electrode handle 30R includes a right-hand current electrode X4 and a right-hand voltage electrode Y4. For measurements of bioelectrical impedances, the human subject stands on the platform 20 and grips the electrode handles 30L and 30R with the left and right hands, respectively, with the arms extending downward.

Under control by the CPU 170, the electrode switching circuit 251 selects two voltage electrodes from among the four voltage electrodes Y1 through Y4 whereas the electrode switching circuit 252 selects two current electrodes from among the four current electrodes X1 through X4, so that impedances Z at various body regions can be determined.

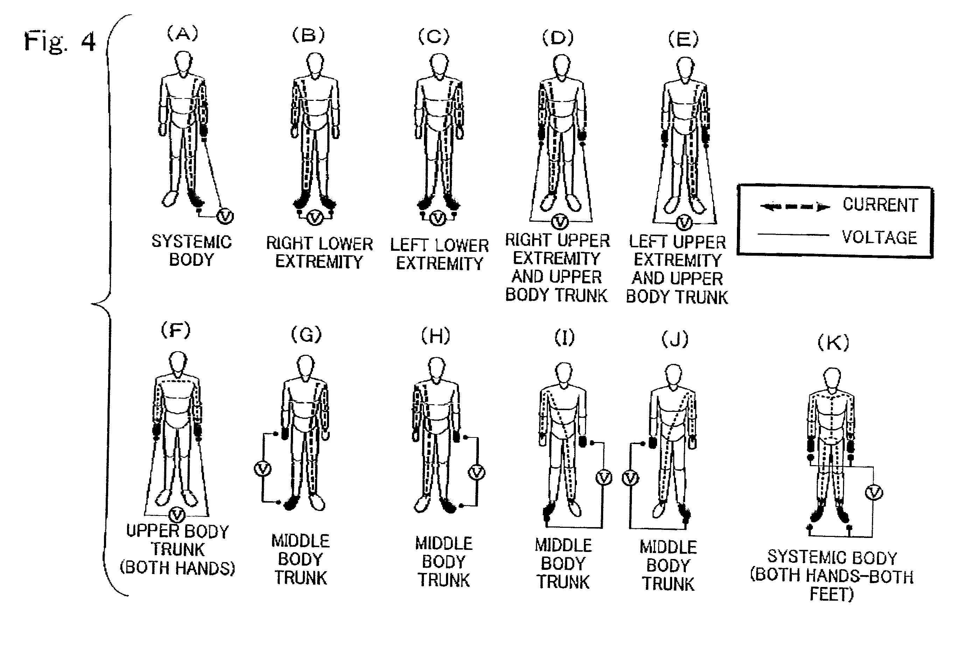

More specifically, as shown in part (A) of Fig. 4, when the reference current $I_{ref}$ is supplied to flow between the left-foot current electrode X1 and the left-hand current electrode X3 and when the potential difference between the left-foot voltage electrode Y1 and the left-hand voltage electrode Y3 is measured, the systemic body bioelectrical impedance can be determined. Although not illustrated, when the reference current $I_{ref}$ is supplied to flow between the right-foot current electrode X2 and the right-hand current electrode X4 and when the potential difference between the right-foot voltage electrode Y2 and the right-hand voltage electrode Y4 is measured, the systemic body bioelectrical impedance can also be determined.

As shown in part (K) of Fig. 4, when the current is supplied to all of the four current electrodes so that a current flows between both hands and a current flows between both feet, and when the potential difference between a hand and a foot is measured, the systemic body bioelectrical impedance can also be determined.

As shown in part (B) of Fig. 4, when the reference current $I_{ref}$ is supplied to flow between the right-foot current electrode X2 and the right-hand current electrode X4 and when the potential difference between the right-foot voltage electrode Y2 and the left-foot voltage electrode Y1 is measured, the bioelectrical impedance at the right lower extremity can be determined.

As shown in part (C) of Fig. 4, when the reference current $I_{ref}$ is supplied to flow between the left-foot current electrode X1 and the left-hand current electrode X3 and when the potential difference between the left-foot voltage electrode Y1 and the right-foot voltage electrode Y2 is measured, the bioelectrical impedance at the left lower extremity can be determined.

As shown in part (D) of Fig. 4, when the reference current $I_{ref}$ is supplied to flow between the right-hand current electrode X4 and the right-foot current electrode X2 and when the potential difference between the right-hand voltage electrode Y4 and the left-hand voltage electrode Y3 is measured, the bioelectrical impedance at the right upper extremity and the upper body trunk can be determined.

As shown in part (E) of Fig. 4, when the reference current $I_{ref}$ is supplied to flow between the left-hand current electrode X3 and the left-foot current electrode X1 and when the potential difference between the right-hand voltage electrode Y4 and the left-hand voltage electrode Y3 is measured, the bioelectrical impedance at the left upper extremity and the upper body trunk can be determined.

As shown in part (F) of Fig. 4, when the reference current $I_{ref}$ is supplied to flow between the right-hand current electrode X4 and the left-hand current electrode X3 and when the potential difference between the right-hand voltage electrode Y4 and the left-hand voltage electrode Y3 is measured, the bioelectrical impedance at the both upper extremities and the upper body trunk can be determined.

As will be understood from each of parts (D), (E), and (F), the determined bioelectrical impedance is influenced by the bioelectrical impedance at the upper body trunk.

As shown in part (G) of Fig. 4, when the reference current $I_{ref}$ is supplied to flow between the left-hand current electrode X3 and the left-foot current electrode X1 and when the potential difference between the right-hand voltage electrode Y4 and the right-foot voltage electrode Y2 is measured, the bioelectrical impedance at the middle body trunk can be determined.

As shown in part (H) of Fig. 4, when the reference current $I_{ref}$ is supplied to flow between the right-hand current electrode X4 and the right-foot current electrode X2 and when the potential difference between the left-hand voltage electrode Y3 and the left-foot voltage electrode Y1 is measured, the bioelectrical impedance at the middle body trunk can be determined.

As shown in part (I) of Fig. 4, when the reference current $I_{ref}$ is supplied to flow between the right-hand current electrode X4 and the left-foot current electrode X1 and when the potential difference between the left-hand voltage electrode Y3 and the right-foot voltage electrode Y2 is measured, the bioelectrical impedance at the middle body trunk can be determined.

As shown in part (J) of Fig. 4, when the reference current $I_{ref}$ is supplied to flow between the right-foot current electrode X2 and the left-hand current electrode X3 and when the potential difference between the right-hand voltage electrode Y4 and the left-foot voltage electrode Y1 is measured, the bioelectrical impedance at the middle body trunk can be determined.

The manner for determining the bioelectrical impedance at the upper or middle body trunk is not limited to the above-described manner. For example, bioelectrical impedances at various regions, e.g., upper and lower extremities, and the systemic body are determined by suitably selecting the current electrodes through which the reference current $I_{ref}$ is supplied and by suitably selecting the voltage electrodes for measuring the potential difference. Then, the bioelectrical impedance at the upper or middle body trunk can be obtained by addition or subtraction of the bioelectrical impedances.

In addition, if electrodes are brought into contact with the ear lobes instead of extremities, the bioelectrical impedance at the body trunk can be obtained. Alternatively, if electrodes are brought into contact with the body trunk directly, the bioelectrical impedance at the upper or middle body trunk can be obtained.

OPERATION

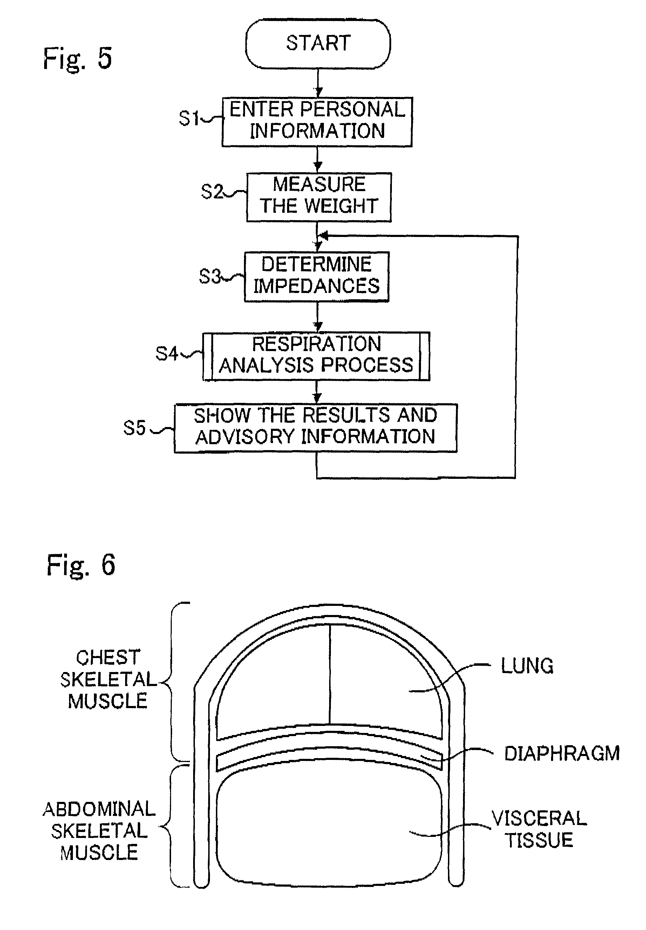

Fig. 5 is a flow chart showing an operation of the body condition determination apparatus 1. Once the power switch is turned on, the power source circuit (not shown) provides power to eclectic elements. Then, the CPU 170 causes the display device 160 to show a screen for facilitating the user to enter personal or individual body information (including the height, age, and sex) at step S1.

After the human interface 150 inputs the personal information into the CPU 170, the CPU 170 causes the weighing scale 110 to measure the weight, and obtains the weight from the weighing scale 110 at step S2.

The CPU 170 causes the bioelectrical impedance determination part 200 to measure the voltages and the currents, each of which is influenced by bioelectrical impedances at desired regions (for example, the extremities and the body trunk), and determines the impedances at the desired body regions at step S3. Thus, the CPU 170 and the bioelectrical impedance determination part 200 serve as a bioelectrical impedance determiner for determining bioelectrical impedance at the desired body regions. Next, the CPU 170 executes a respiration analysis process at step S4. As will be described later in detail, in the respiration analysis process, the type of respiration is analyzed and the degrees of abdominal respiration and costal respiration are analyzed. Thus, the CPU 170 serves as a respiration type determiner for determining whether respiration of the human subject is abdominal or costal on the basis of change over time of the bioelectrical impedance.

Then, the CPU 170 causes the display device 160 to show the results of the respiration analysis process and advisory information at step S5.

In order to analyze change over time of impedance, the CPU 170 returns the operation to step S3. Although not shown, the operation may be ended by the user's instruction.

PRINCIPLES OF RESPIRATION ANALYSIS

The principles of respiration analysis will be described. Fig. 6 is a schematic view showing tissues in the body trunk. As shown in Fig. 6, tissues in the body trunk are separated by the diaphragm into the upper and lower parts. The upper part includes the lungs and the chest skeletal muscle including the internal and external intercostal muscles. On the other hand, the lower part includes the visceral tissue and the abdominal skeletal muscle including the internal and external abdominal oblique muscles, the transverse abdominal muscle, and the abdominal rectus muscle.

In both of abdominal respiration and costal respiration, the diaphragm moves up to compress the lungs at exhalation and moves down to expand the lungs at inhalation. The characteristic of abdominal respiration that does not appear in costal respiration is that the visceral tissue expands and contracts in the perpendicular direction so as to move up and down the diaphragm due to ventrodorsal contraction and expansion of the abdominal respiratory muscle including the abdominal rectus muscle, the internal and external abdominal oblique muscles, and the transverse abdominal muscle.

When muscles or tissues move, impedances at the corresponding regions change. The aspect of change of the bioelectrical impedance at a body region in abdominal respiration is different from that in costal respiration. Accordingly, on the basis of change over time of the bioelectrical impedance at a body region of a human subject that contributes to respiration which is costal respiration or abdominal respiration, it is possible to determine the type of respiration of the human subject.

Respiration of human beings includes a complete respiration in addition to costal respiration and abdominal respiration. During complete respiration, the human performs costal respiration and abdominal respiration simultaneously.

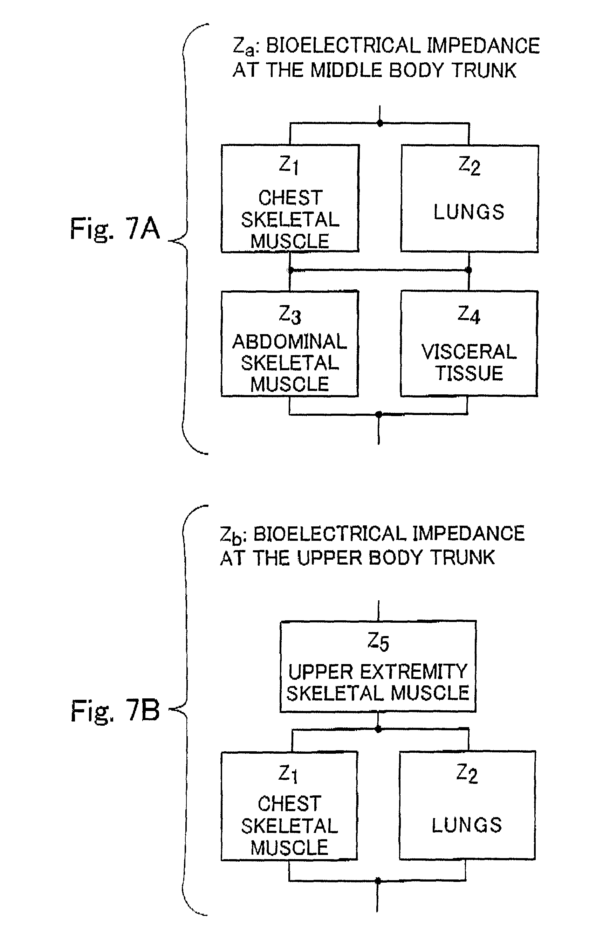

With reference to the equivalent circuit models in Figs. 7A and 7B, influential factors for the bioelectrical impedance $Z_a$ at the middle body trunk and the bioelectrical impedance $Z_b$ at the upper body trunk will be described. Let us assume that the bioelectrical impedance at the chest skeletal muscle is $Z_1$, that at the lungs is $Z_2$, that at the abdominal skeletal muscle is $Z_3$, that at the visceral tissue is $Z_4$, and that at the upper extremity skeletal muscle is $Z_5$. The bioelectrical impedance $Z_4$ at the visceral tissue includes the bioelectrical impedance of the diaphragm. As will be understood from Fig. 7A, the bioelectrical impedance $Z_a$ (second bioelectrical impedance) at the middle body trunk (second region) is influenced by impedances $Z_1$ and $Z_2$ in parallel with each other and by impedances $Z_3$ and $Z_4$ in parallel with each other. As will be understood from Fig. 7B, the bioelectrical impedance $Z_b$ (first bioelectrical impedance) at the upper body trunk (first region) is influenced by impedances $Z_1$ and $Z_2$ in parallel with each other and by impedance $Z_5$.

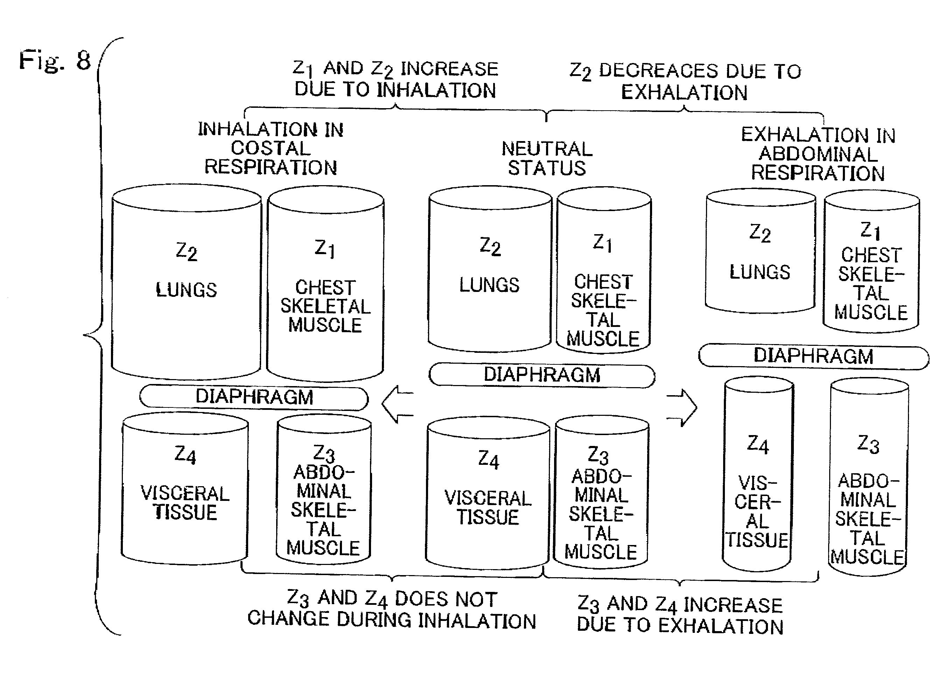

With reference to Fig. 8, relationship between respiration and change of bioelectrical impedance will be described.

Change in the impedance $Z_b$ (first bioelectrical impedance) at the upper body trunk (first region) in respiration is mainly caused by air entering and exiting the lungs, which has a high electrical insulation property and causes change in the electrical characteristic (i.e., the electrical conductivity or the inverse of the volume resistivity) at the upper body trunk. During exhalations (expirations), the bioelectrical impedance $Z_2$ at the lungs decreases due to reduction in the volume of air inside tissues in the lungs. During inhalations (inspirations), the

bioelectrical impedance $Z_2$ at the lungs increases due to increase in the volume of air inside tissues in the lungs.

In costal respiration, which expands and contracts the thoracic cage, the chest skeletal muscle that contributes to respiration (e.g., internal and external intercostal muscles) expands and contracts in a similar way to the expansion and contraction of the lungs. Therefore, in costal respiration, when the lungs expand so that the bioelectrical impedance $Z_2$ at the lungs increases, the chest skeletal muscle also expands, and the bioelectrical impedance $Z_1$ at the chest skeletal muscle also increases. Similarly, when the lungs contract so that the bioelectrical impedance $Z_2$ at the lungs decreases, the chest skeletal muscle also contracts and the bioelectrical impedance $Z_1$ at the chest skeletal muscle also decreases.

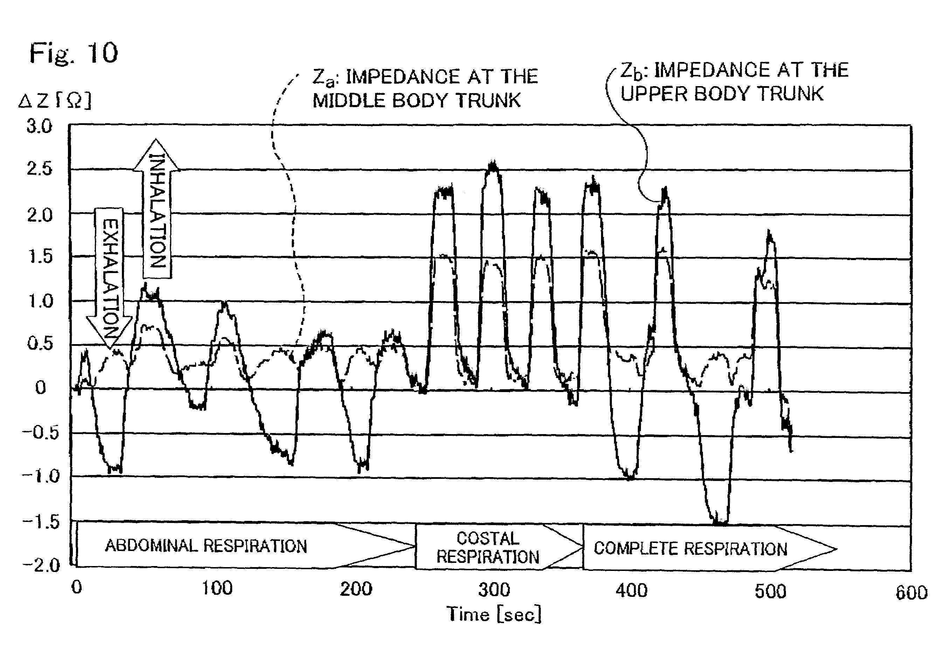

However, in abdominal respiration in which the thoracic cage does not change in volume significantly, although the bioelectrical impedance $Z_2$ at the lungs changes significantly due to respiration, the bioelectrical impedance $Z_1$ at the chest skeletal muscle does not change significantly. Therefore, the amplitude of change in the bioelectrical impedance $Z_b$ at the upper body trunk in abdominal respiration is slightly less than that in costal respiration, as shown in Fig. 10.

Change in the impedance $Z_a$ (second bioelectrical impedance) at the middle body trunk (second region) in respiration relates to movement of the diaphragm. As described above, in both abdominal respiration and costal respiration, the diaphragm moves up to compress the lungs at exhalation and moves down to expand the lungs at inhalation. The characteristic of abdominal respiration that does not appear in costal respiration is that the visceral tissue expands and contracts in the perpendicular direction for moving up and down the diaphragm due to ventrodorsal contraction and expansion of the abdominal respiratory muscle (abdominal skeletal muscle).

More specifically, in abdominal respiration, during exhalations, the human subject contracts the abdominal muscle ventrodorsally so as to move up the diaphragm together. As a result, the visceral tissue and the abdominal skeletal muscle expand in the perpendicular direction, thereby increasing the impedance $Z_3$ at the abdominal skeletal muscle and the impedance $Z_4$ at the visceral tissue. During exhalations, the bioelectrical impedance $Z_2$ at the lungs decreases due to reduction in the volume of air inside tissues in the lungs as described above. Therefore, in abdominal respiration, when the bioelectrical impedance at the chest region above the diaphragm decreases, the bioelectrical impedance at the abdominal region beneath the diaphragm increases. In other words, the bioelectrical impedance of the abdominal region changes in an opposite manner to the change of the bioelectrical impedance at the chest region when the human subject performs exhalation in abdominal respiration. The amplitude of change in the bioelectrical impedance $Z_a$ at the middle body trunk in abdominal respiration is remarkably less than that in costal respiration, as shown in Fig. 10. During exhalations in abdominal respiration, usually, the increment of bioelectrical impedance at the abdominal region beneath the diaphragm (the visceral tissue and the abdominal skeletal muscle) is less than the decrement of bioelectrical impedance at the chest region above the diaphragm (the lungs and the chest skeletal muscle), the resulting bioelectrical impedance $Z_a$ at the middle body trunk decreases in an opposite manner to the bioelectrical impedance $Z_b$ at the upper body trunk.

During exhalations in abdominal respiration, if the increment of bioelectrical impedance at the abdominal region beneath the diaphragm (the visceral tissue and the abdominal skeletal muscle) is in excess of the decrement of bioelectrical impedance at the chest region above the diaphragm (the lungs and the chest skeletal muscle), the resulting bioelectrical impedance $Z_a$ at the middle body trunk increases. This exceptional case occurs when a human subject has very little visceral fat or is skilled in abdominal respiration.

If a human subject has excessive visceral fat mass, the abdominal muscle will not contract and the visceral tissue will not expand perpendicularly. Accordingly, the resulting bioelectrical impedance $Z_a$ at the middle body trunk does not change similarly to the bioelectrical impedance $Z_b$ at the upper body trunk. It is expected that humans with excessive visceral fat mass usually perform costal respiration and perform abdominal respiration less often.

As will be understood from the above description, the movement of the abdominal region (including abdominal skeletal muscle and the visceral tissue) beneath the diaphragm varies depending on the type of respiration (costal and abdominal respirations). If change of the abdominal bioelectrical impedance with the passage of time is traced, it is possible to determine the type of respiration of the human subject. In this embodiment, the difference between the bioelectrical impedance $Z_b$ at the upper body trunk and the bioelectrical impedance $Z_a$ at the middle body trunk is calculated as the abdominal bioelectrical impedance, and respiration of the human subjects is analyzed on the basis of the calculated difference. As will be understood from Figs. 7A and 7B, the impedance difference $\Delta Z$ is represented as follows:

$$\Delta Z = Z_b - Z_a$$
$$= Z_5 - Z_3 \times Z_4 / (Z_3 + Z_4)$$

The bioelectrical impedance $Z_b$ at the upper body trunk is influenced by the bioelectrical impedance $Z_5$ at the upper extremity skeletal muscle as shown in Fig. 7B. The upper extremity skeletal muscle does not directly contribute to respiration. In view of the accuracy of determination, it is undesirable that the upper extremity skeletal muscle move during determination of impedances. In this embodiment, during determination of impedances, each human subject stands on the platform 20 and grips the electrode handles 30L and 30R with the left and right hands, respectively, with the arms extending downward. Therefore, the upper extremity skeletal muscle does not move during determination of impedances, and change over time of the resulting impedance difference $\Delta Z$ reflects accurately the type of respiration.

RESPIRATION ANALYSIS PROCESS

Two examples of the aforementioned respiration analysis process will be described.

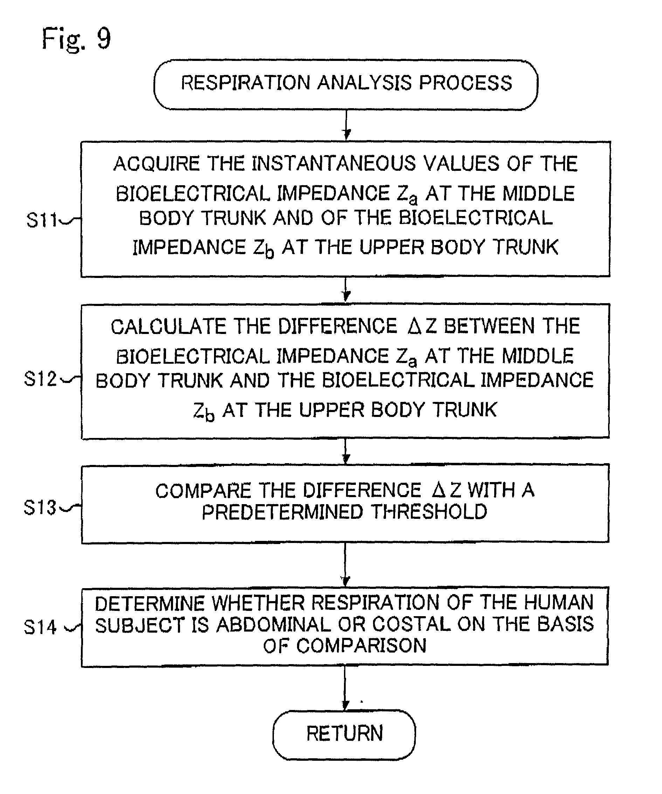

Fig. 9 shows a first example of the respiration analysis process. First, the CPU 170 acquires, at step S11, the instantaneous value of the bioelectrical impedance $Z_a$ at the middle body trunk and the instantaneous value of the bioelectrical impedance $Z_b$ at the upper body trunk that were determined at step S3. The bioelectrical impedance $Z_a$ at the middle body trunk and the bioelectrical impedance $Z_b$ at the upper body trunk change for example as shown in Fig. 10.

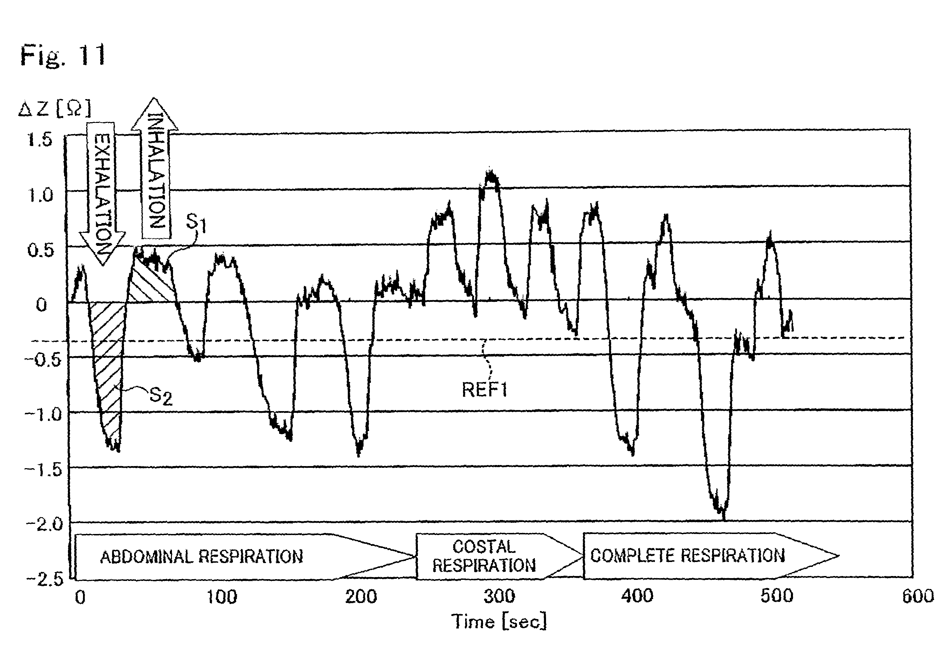

Next, the CPU 170 serves as a difference calculator for calculating the impedance difference $\Delta Z$ between the bioelectrical impedance $Z_a$ at the middle body trunk and the bioelectrical impedance $Z_b$ at the upper body trunk at step S12. The impedance difference $\Delta Z$ corresponds to the abdominal bioelectrical impedance. The impedance difference $\Delta Z$ changes as shown in Fig. 11.

Then, the CPU 170 serves as a comparer for comparing the instantaneous impedance difference $\Delta Z$ with a predetermined threshold at step S 13. The CPU 170 (respiration type determiner) determines whether respiration of the human subject is abdominal or costal on the basis of the comparison at step S14.

For example, if the threshold REF1 is set as shown in Fig. 11, exhalations in abdominal respiration can be appropriately detected. In addition, as shown in Fig. 11, the impedance difference $\Delta Z$ is continuously in excess of the threshold REF1 during costal respiration.

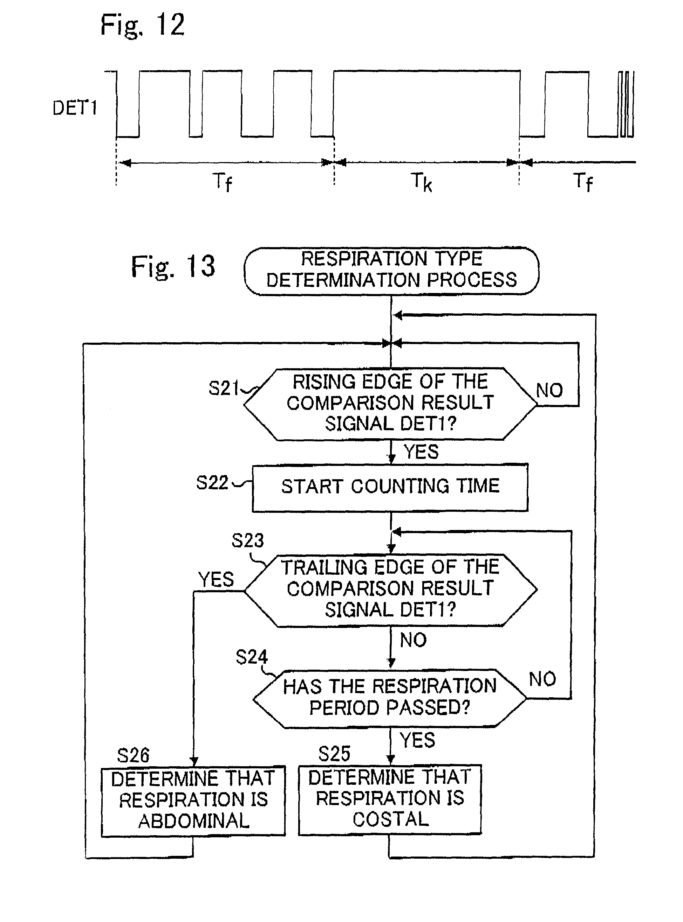

Fig. 12 shows change over time of a comparison result signal DET1 produced by the CPU 170. When the impedance difference $\Delta Z$ is beneath the threshold REF1, the CPU makes the comparison result signal DET1 at the low level. When the impedance difference $\Delta Z$ is over the threshold REF1, the CPU makes the comparison result signal DET1 at the high level, During exhalations in abdominal respiration, the comparison result signal DET1 is at the low level. The period $T_f$ in Fig. 12 in which the comparison result signal DET1 rises and falls in a short cycle can be considered as a period for abdominal respiration. The period $T_k$ in Fig. 12 in which the comparison result signal DET1 continues at the high level for a long period can be considered as a period for costal respiration.

Thus, the comparison result signal DET1 changes in a short cycle in abdominal respiration, whereas the comparison result signal DET1 changes in a long cycle in costal respiration. In this embodiment, the boundary between "short" and "long" is determined on the basis of a normal respiration cycle or period of each human subject. More specifically, when the comparison result signal DET1 rises and falls within the normal respiration period, the CPU 170 determines that the respiration is abdominal. When the rise and fall of the comparison result signal DET1 takes a period longer than the normal respiration period, the CPU 170 determines that the respiration is costal. Using the normal respiration period as the base for the determination is reasonable since exhalations and inhalations alternate.

Fig. 13 shows an example of a respiration type determination process that corresponds to step S14 in Fig. 9. At step S21, the CPU 170 decides whether or not a rising edge of the comparison result signal DET1 has been detected. The CPU 170 repeats the decision until a rising edge is detected. The rising edge of the comparison result signal DET1 corresponds to the time point at which the impedance difference $\Delta Z$ rises above the threshold REF1 due to inhalation. Next, the CPU 170 starts counting elapsed time by using a counter at step S22. Thus, the elapsed time is counted from the approximate start of the inhalation.

At step S23, the CPU 170 decides whether or not a trailing edge of the comparison result signal DET1 has been detected. The trailing edge of the comparison result signal DET1 corresponds to the time point at which the impedance difference $\Delta Z$ drops below the threshold REF1 due to exhalation. If the decision at step S23 is negative, the process proceeds to step S24, at which the CPU 170 refers to the elapsed time counted by the counter and decides whether or not the respiration period has passed from the start of the inhalation. The decision at step S24 is affirmative if the impedance difference $\Delta Z$ has not dropped below the threshold REF1 although the respiration period has passed (the human subject has exhaled after the approximate start of the inhalation). If the decision at step S24 is affirmative, the CPU 170 determines that the human subject is performing costal respiration at step S25, and returns the process to step S21.

On the other hand, if the decision at step S24 is negative, the CPU 170 returns the process to step S23. If the trailing edge of the comparison result signal DET1 has been detected before the elapse of the respiration period after the last rising edge of the comparison result signal DET1, the decision at step S23 is affirmative. In this case, the CPU 170 determines that the human subject is performing abdominal respiration at step S26, and returns the process to step S21. Although not shown, the respiration type determination process may be ended by the user's instruction.

The respiration period used as step S24 as the base for decision may be determined by measuring a standard respiration period of the human subject at rest composed of an exhalation period and an inhalation period. Alternatively, the respiration period may be determined by measuring a standard cycle of variation of the bioelectrical impedance $Z_b$ (first bioelectrical impedance) at the upper body trunk since the bioelectrical impedances $Z_1$ and $Z_2$ change cyclically due to breathing in both of costal respiration and abdominal respiration. The CPU 170 may serve as a respiration period decider that determines the respiration period.

As shown in Figs. 10 and 11, respiration of human beings in fact includes a complete respiration in addition to costal respiration and abdominal respiration. During complete respiration, the human performs costal respiration and abdominal respiration simultaneously. In the above-described respiration analysis process, the period during which abdominal respiration is mainly performed can be detected as the period for abdominal respiration, and the period for complete respiration can also be detected as the period for abdominal respiration. The period during which costal respiration is mainly performed can be detected as the period for costal respiration.

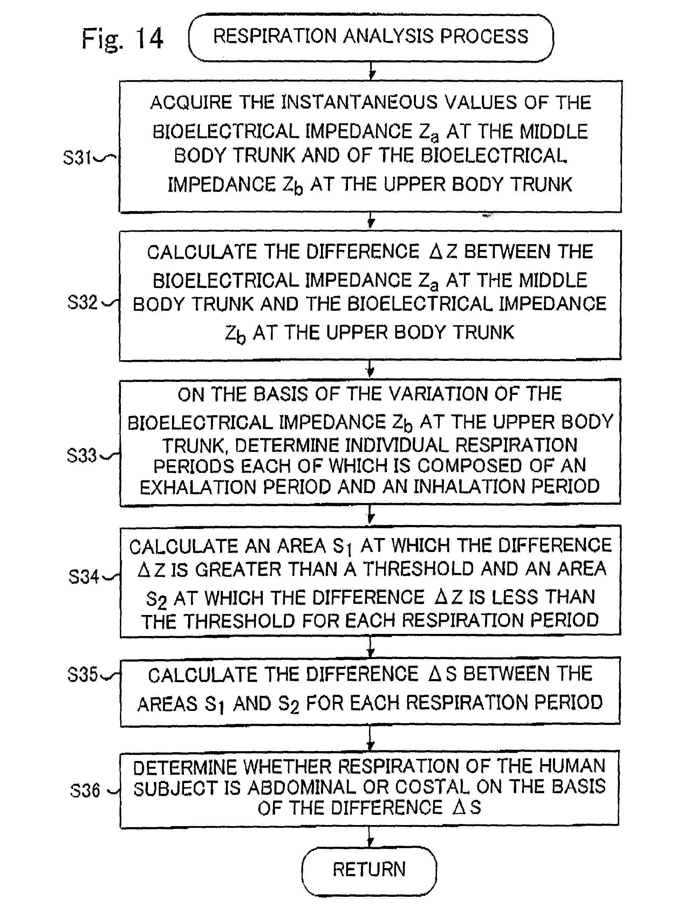

A second example of the respiration analysis process will be described with reference to Fig. 14. First, the CPU 170 acquires, at step S31, the instantaneous value of the bioelectrical impedance $Z_a$ at the middle body trunk and the instantaneous value of the bioelectrical impedance $Z_b$ at the upper body trunk that were determined at step S3. Next, the CPU 170 calculates the impedance difference $\Delta Z$ between the bioelectrical impedance $Z_a$ at the middle body trunk and the bioelectrical impedance $Z_b$ at the upper body trunk at step S32. As described above, the impedance difference $\Delta Z$ corresponds to the abdominal bioelectrical impedance and changes as shown in Fig. 11.

At step S33, on the basis of the variation of the bioelectrical impedance $Z_b$ (first bioelectrical impedance) at the upper body trunk, the CPU 170 serves as a respiration period decider that determines individual respiration periods of the human subject, each of which is composed of an exhalation period and an inhalation period. The determination of individual respiration periods may be realized by comparing the variation of the bioelectrical impedance $Z_b$ (first bioelectrical impedance) at the upper body trunk with a predetermined threshold, and obtaining the cycle of variation of bioelectrical impedance $Z_b$.

At step S34, the CPU 170 calculates an area $S_1$ at which the impedance difference $\Delta Z$ is greater than a threshold and an area $S_2$ at which the impedance difference $\Delta Z$ is less than the threshold for each respiration period. In other words, the CPU 170 serves as an integrator for integrating the impedance difference $\Delta Z$ over the threshold to produce a first integral of the impedance difference $\Delta Z$ in an inhalation, and for integrating the impendance difference $\Delta Z$ beneath the threshold to produce a second integral of the impedance difference $\Delta Z$ in an exhalation, for each respiration period. If the threshold is for example zero ohms in Fig. 11, the areas $S_1$ and $S_2$ are as in Fig. 11.

The CPU 170 calculates the difference $\Delta S$ be-

tween the areas $S_1$ and $S_2$ (i.e., integral difference between the first integral of the impedance difference $\Delta Z$ in an inhalation and the second integral of the impedance difference $\Delta Z$ in an exhalation) for each respiration period at step S35. That is, the CPU 170 serves as an integrator for deriving an integration $\Delta S$ of the impedance difference $\Delta Z$. The CPU 170 (respiration type determiner) determines whether respiration of the human subject is abdominal or costal on the basis of the difference $\Delta S$ at step S36. Since the impedance difference $\Delta Z$ corresponds to the abdominal bioelectrical impedance at the abdominal region as described above, the CPU 170 (respiration type determiner) determines whether respiration of the human subject is abdominal or costal on the basis of difference $\Delta S$ between change over time of the abdominal bioelectrical impedance ($\Delta Z$) in an inhalation of the human subject and change over time of the abdominal bioelectrical impedance ($\Delta Z$) in an exhalation of the human subject.

As will be understood from the above, $\Delta S = S_1 - S_2$. Let us assume that the difference $\Delta S$ in abdominal respiration is defined as $\Delta S_{abd}$, the difference $\Delta S$ in costal respiration is defined as $\Delta S_{cos}$, and the difference $\Delta S$ in complete respiration is defined as $\Delta S_{con}$. The inventor found the following relationship:

$$\Delta S_{abd} < \Delta S_{con} < \Delta S_{cos}.$$

The difference $\Delta S$ can be used for an index indicative of the degree of abdominal respiration and the degree of costal respiration in respiration of the human subject. On the basis of the difference $\Delta S$, it is possible to determine the type of respiration and to determine the degrees of costal or abdominal respirations.

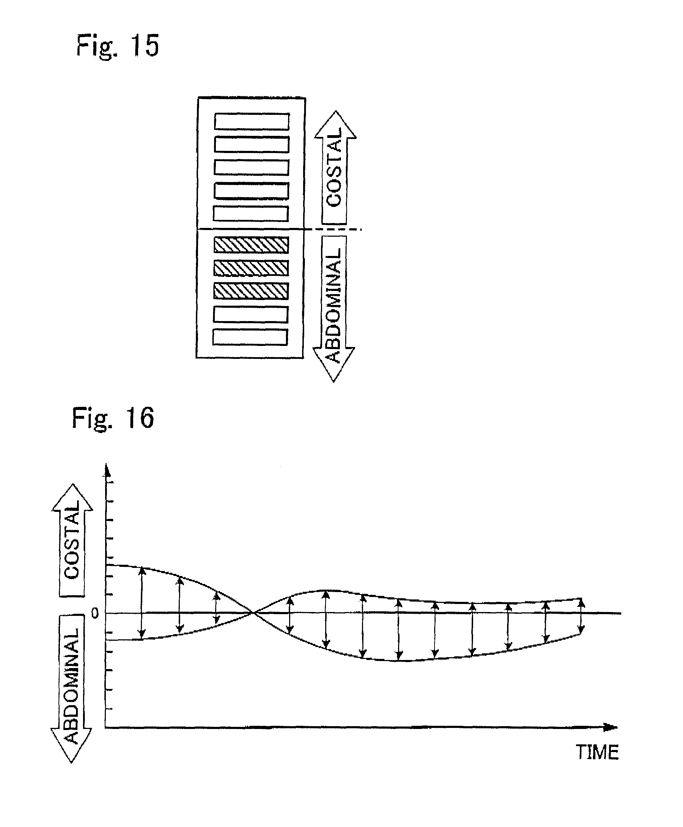

As shown in Fig. 15, the CPU 170 may cause the display device 160 (first reporter) to show degrees of abdominal respiration and costal respiration. The example shown in Fig. 15 is a bar graph image that indicates the degree of the present respiration in real time. The user (the person performing the monitoring or the human subject) can know the direction as to which of costal respiration and abdominal respiration is more prevailing within the present respiration and the degree of the present respiration. Alternatively, such a bar graph image may indicate the depth of respiration.

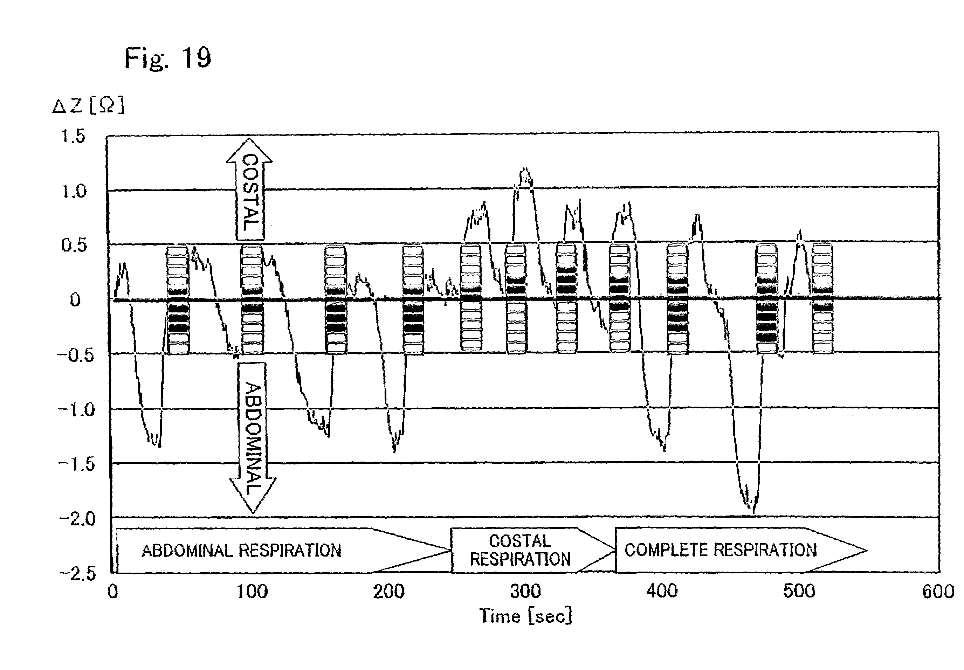

The CPU 170 may cause the display device 160 (first reporter) to change the bar graph image shown on the display device 160 as time advances, for example, as shown in Fig. 19. In this example, the positive direction related to positive values of the impedance difference $\Delta Z$ above the aforementioned threshold REF1 means costal respiration, whereas the negative direction means abdominal respiration. The bar graph image changes for each respiration. The user (the person performing the monitoring or the human subject) can know of the changes in degrees of abdominal respiration and costal respiration in real time.

In this example of the bar graph image, a bar corresponds to an extent of the impedance difference $\Delta Z$, e.g., zero to 0.5 ohms. The bar graph image includes five bars for costal respiration and five bars for abdominal respiration, so as to be capable of indicating five degrees for each of costal respiration and abdominal respiration.

The user can understand the type of respiration with the bar graph image. For example, if only a single bar (a degree) is highlighted (i.e., the impedance difference $\Delta Z$ is zero to 0.5 ohms), the respiration cannot be classified into costal or abdominal. If two or more bars at one side are highlighted, the respiration can be classified as costal or abdominal. If two or more bars at the costal respiration side are highlighted and one or zero bars at the abdominal respiration side are highlighted, the respiration can be classified as costal. In contrast, if two or more bars at the abdominal respiration side are highlighted and one or zero bars at the costal respiration side are highlighted, the respiration can be classified as abdominal respiration. If two or more bars at both of the abdominal and costal respiration sides are highlighted, the respiration can be classified as complete. This classification is made by the user, but the same classification scheme may also be conducted at the CPU 170 for the respiration type determination process instead of that shown in Fig. 13.

As described above, the abdominal bioelectrical impedance is calculated as the impedance difference $\Delta Z$ between the bioelectrical impedance $Z_a$ at the middle body trunk and the bioelectrical impedance $Z_b$ at the upper body trunk for determining the type of respiration in this embodiment. In this embodiment, it is possible to monitor the changes in respiration at the body trunk, and to determine the type of respiration and the degree of respiration, with the use of electrodes to be arranged at hands and feet which are also widely used for determining body compositions and without arranging electrodes or strain gauges at the body trunk.

VARIATIONS AND MODIFICATIONS

The present invention is not limited to the above described embodiment. For example, various modifications can be made as follows:

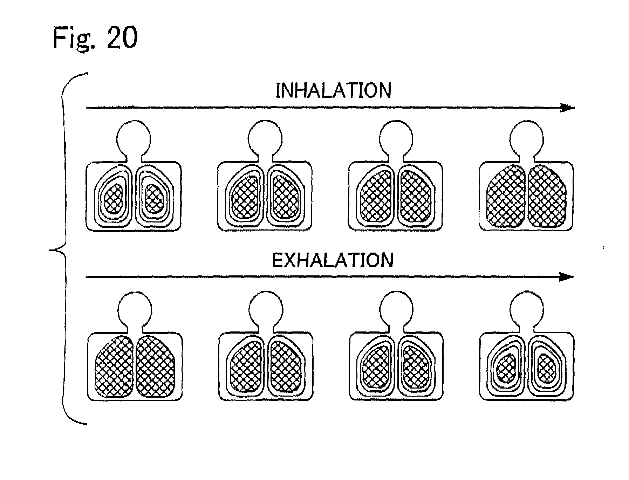

In the above described embodiment, the bar graph image is used for output information. However, another type of output information may be used. For example, as shown in Fig. 20, depth of respiration can be represented by a schematic movie of the lungs. In the schematic movie, as time passes in each inhalation, the highlighted area in the lungs increases. In addition, as time passes in each exhalation, the highlighted area in the lungs decreases. This schematic movie is used for output information based on the determination result. However, this schematic movie is also used for guidance in respiration rhythm.

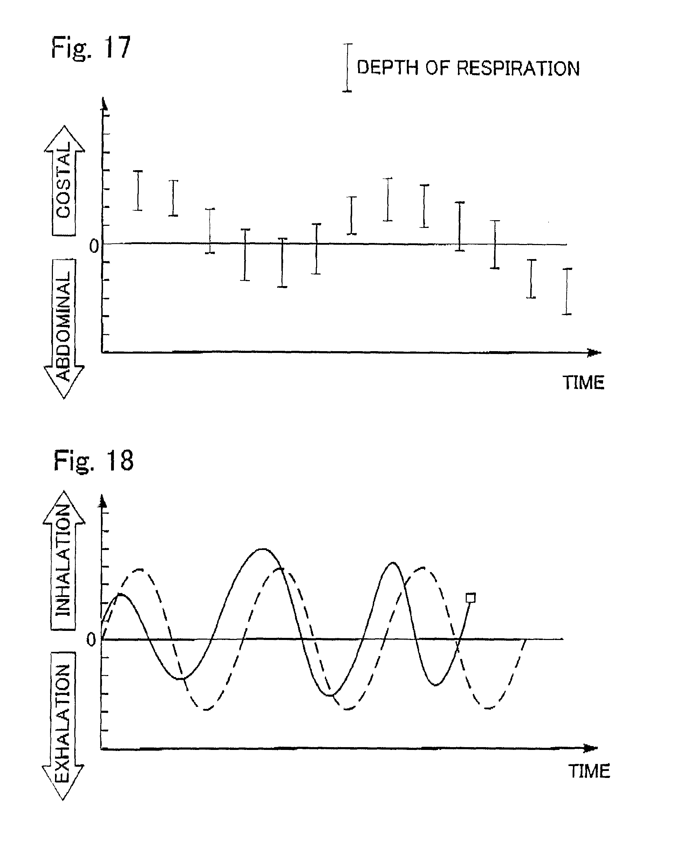

As shown in Fig. 16, the CPU 170 may cause the display device 160 (first reporter) to show a sequential line graph in which the change over time of each of the degrees of costal respiration and abdominal respiration. Alternatively, as shown in Fig. 17, the CPU 170 may cause the display device 160 (second reporter) to show changes over time in the depth of respiration with the degree of costal or abdominal respiration. Displaying the degrees of abdominal respiration and costal respiration in time series can be utilized as useful biofeedback information for respiration training.

In addition to displaying change of the degrees of abdominal respiration and costal respiration, if respiration guidance information is displayed, it is possible to inform the human subject of the difference between ideal respiration and actually measured respiration. In this case, biofeedback effects can be further enhanced. For example, as shown in Fig. 18, the CPU 170 may cause the display device 160 (second reporter) to show change of the depth of exhalation and inhalation. In this example, the ideal respiration is denoted by dotted line, whereas the actually measured respiration is denoted by the solid line.

In addition to, or instead of, the real time display of the analysis, the CPU 170 may analyze data accumulated in a sampling period including a plurality of respiration cycles, and may cause the display device 160 to show the determination result as to whether costal or abdominal respiration is prevailing.

The CPU 170 may cause the speaker 145 to make background sounds for the human subject to relax (voices, music, or natural sounds) in addition to sounding a message for respiration guidance, for example, guidance in respiration rhythm.

In the above described embodiment, four current electrodes and four voltage electrodes are arranged at both hands and both feet in accordance with the limb-lead eight-electrode method. The present invention is not limited to the embodiment. For example, the bioelectrical impedance at the upper body trunk can be measured with the use of a combination of the limb-lead method and ear electrodes. By using ear electrodes, the bioelectrical impedance at the upper body trunk is influenced by only one upper extremity rather than both upper extremities. If ear electrodes are incorporated in earphones or headphones, further advantages are obtained since sound information and relaxing effects are given to the human subject.

In the above-described embodiment, impedances are determined when the human subject is standing. However, impedances may be determined when the human subject is at a sitting or a relaxed position on a sofa, a seat, e.g., a lavatory seat, or a chair, e.g., a massage chair, with electrodes being arranged at parts of the seat, armrests, footrests, or a combination thereof.

Furthermore, impedances are determined when the human subject is bathing with electrodes being arranged at armrests and the bottom surface of the bathtub at which the buttocks and soles of the human subject are in contact. The body trunk includes physiological saline and is electrically more conductive than the hot water in a bathtub. Accordingly, when the human subject is relaxed while bathing, impedances are determined and respiration training may be done.

The body condition determination apparatus 1 of the above-described embodiment may include a blood-pressure meter with a cuff. Change of the status of respiration or the tension on the arms is determined on the basis of the determined impedances, and obtained information may be used for correcting or compensating the measured blood pressure.

In the above-described embodiment and variations, the maximum and minimum of bioelectrical impedance at each of the middle body trunk and the upper body trunk for each respiration period may be obtained. On the basis of the maximum and minimum, it is possible to calculate the amplitude of bioelectrical impedance at each of the middle body trunk and the upper body trunk, or the difference of the amplitude. The body condition determination apparatus may execute the respiration analysis with the use of at least one of the amplitude or the difference of the amplitude.

In the above-described embodiment and variations, change of the determined bioelectrical impedance may be normalized to the average impedance or the median impedance over a sampling period or a respiration period.

The CPU 170 may correct or compensate at least one of the bioelectrical impedances at the middle body trunk and the upper body trunk, so as to enlarge the impedance difference $\Delta Z$. Therefore, the difference calculator for calculating the impedance difference $\Delta Z$ may include a compensator for compensating at least one of the first bioelectrical impedance or the second bioelectrical impedance, and the difference calculator calculates the impedance difference $\Delta Z$ between the first bioelectrical impedance and the second bioelectrical impedance among which at least one is compensated by the compensator. In this specification, the term "compensate" includes "normalize". Therefore, the compensator may normalize change of the first or second bioelectrical impedance to an averaged or median value over a sampling period or a respiration period.

The CPU 170 (compensator) may correct or compensate at least one of the bioelectrical impedances at the middle body trunk and the upper body trunk on the basis of personal or individual body indexes or parameters, such as the age, height, weight, or body fat ratio of the human subject. In this case, in light of variation of details of body compositions, for example, distribution of skeletal muscles in the body trunk or the upper extremities, reliability of the determined type of respiration or the degree or depth of respiration is enhanced.

In the above-described embodiment, bioelectrical impedances at two body regions, i.e., the upper body trunk (first region) and the middle body trunk (second region) are determined, and it is determined, on the basis of change of the difference therebetween over time, as to whether respiration of the human subject is abdominal or costal. However, the present invention is not limited to the embodiment. The body condition determination apparatus may determine biological impedances at another first region of the human subject including the lungs and at another second region of the human subject, the second region being specified such that the second bioelectrical impedance changes in an opposite manner to change of the first bioelectrical impedance when the human subject performs exhalation in abdominal respiration. In this case, the bioelectrical impedance at the first region changes similarly in both costal respiration and abdominal respiration, but the bioelectrical impedance at the second region changes differently in costal respiration and abdominal respiration. On the basis of change in the difference between both bioelectrical impedances, it is possible to determine whether respiration of the human subject is abdominal or costal.

The body condition determination apparatus may determine whether respiration of the human subject is abdominal or costal on the basis of change over time of the bioelectrical impedance at the middle body trunk that includes the lungs and the abdomen since the amplitude of change of impedance $Z_a$ at the middle body trunk in abdominal respiration is remarkably less than that in costal respiration, as shown in Fig. 10. For example, if the bioelectrical impedance over zero ohms in each respiration period is integrated, the integration results in abdominal respiration are remarkably less than those in costal respiration. Distinction between abdominal respiration and costal respiration can be made by comparing the integration results with a suitable threshold. Consequently, even if a single body region including a partial region at which the bioelectrical impedance changes similarly in both of costal respiration and abdominal respiration and another partial region at which the bioelectrical impedance changes in an opposite manner in costal respiration and abdominal respiration is selected as the subject of impedance determination, it is possible to determine whether respiration of the human subject is abdominal or costal.

While the present invention has been particularly shown and described with references to preferred embodiments thereof, it will be understood by those skilled in the art that various changes in form and details may be made therein without departing from the scope of the invention as defined by the claims. Such variations, alterations, and modifications are intended to be encompassed in the scope of the present invention.


**Claims**

1. A respiration type evaluation apparatus (1) comprising:

a bioelectrical impedance determiner (170, 200) adapted for determining a bioelectrical impedance ($Z_a$, $Z_b$, $\Delta Z$) at at least a body region of a human subject that contributes to respiration of the human subject; and
a respiration type determiner (170) adapted for determining whether respiration of the human subject is abdominal or costal on the basis of change over time of the bioelectrical impedance ($Z_a$, $Z_b$, $\Delta Z$) determined by the bioelectrical impedance determiner (170, 200).

2. The respiration type evaluation apparatus (1) according to claim 1, wherein the change over time of the bioelectrical impedance ($\Delta Z$) at the body region includes periodical change of the bioelectrical impedance influenced by respirations of the human subject, each including an inhalation and an exhalation, and wherein the respiration type determiner (170) is adapted for determining whether respiration of the human subject is abdominal or costal on the basis of a difference ($\Delta S$) between change over time of the bioelectrical impedance ($\Delta Z$) in an inhalation of the human subject and change over time of the bioelectrical impedance ($\Delta Z$) in an exhalation of the human subject.

3. The respiration type evaluation apparatus (1) according to claim 1, wherein the bioelectrical impedance determiner (170, 200) is adapted for determining a first bioelectrical impedance ($Z_b$) at a first region of the human subject including the lungs and a second bioelectrical impedance ($Z_a$) at a second region of the human subject, the second region being specified such that the second bioelectrical impedance ($Z_a$) changes in an opposite manner to change of the first bioelectrical impedance ($Z_b$) when the human subject performs exhalation in abdominal respiration, and wherein the respiration type determiner (170) is adapted for determining whether respiration of the human subject is abdominal or costal on the basis of a difference ($\Delta Z$) between change over time of the first bioelectrical impedance ($Z_b$) and change over time of the second bioelectrical impedance ($Z_a$).

4. The respiration type evaluation apparatus (1) according to claim 3, wherein the bioelectrical impedance determiner (170, 200) is adapted for determining the first bioelectrical impedance ($Z_b$) at the first region including the lungs of the human subject and excluding the abdomen of the human subject and determines the second bioelectrical impedance ($Z_a$) at the second region including the abdomen of the human subject.

5. The respiration type evaluation apparatus (1) according to claim 3 or 4, wherein the respiration type determiner (170) comprises a difference calculator (170) adapted for calculating an impedance difference ($\Delta Z$) between the first bioelectrical impedance ($Z_b$) and the second bioelectrical impedance ($Z_a$), and wherein the respiration type determiner (170) is adapted for determining whether respiration of the human subject is abdominal or costal on the basis of change over time of the impedance difference ($\Delta Z$).

6. The respiration type evaluation apparatus (1) according to claim 5, wherein the difference calculator (170) comprises a compensator (170) adapted for compensating at least one of the first bioelectrical impendance ($Z_b$) or the second bioelectrical impedance ($Z_a$), and wherein the difference calculator (170) is adapted for calculating the impedance difference ($\Delta Z$) between the first bioelectrical impedance ($Z_b$) and the second bioelectrical impedance ($Z_a$) among which at least one is compensated by the compensator (170).

7. The respiration type evaluation apparatus (1) according to claim 6, wherein the compensator (170) is adapted for normalizing change of the first bioelectrical impedance ($Z_b$) or change of the second bioelectrical impedance ($Z_a$) to an averaged or median value.

8. The respiration type evaluation apparatus (1) according to claim 6, wherein the compensator (170) is adapted for compensating the first bioelectrical impedance ($Z_b$) or the second bioelectrical impedance ($Z_a$) on the basis of personal body indexes.

9. The respiration type evaluation apparatus (1) according to any one of claims 5 to 8, wherein the respiration type determiner (170) comprises a comparer (170) adapted for comparing the impedance difference ($\Delta Z$) with a threshold (REF1), and wherein the respiration type determiner (170) is adapted for determining whether respiration of the human subject is abdominal or costal on the basis of the comparison at the comparer (170).

10. The respiration type evaluation apparatus (1) according to any one of claims 5 to 8, wherein the respiration type determiner (170) comprises an integrator (170) for deriving an integration ($\Delta S$) of the impedance difference ($\Delta Z$), and wherein the respiration type determiner (170) is adapted for determining whether respiration of the human subject is abdominal or costal on the basis of the integration ($\Delta S$) of the impedance difference ($\Delta Z$) derived at the integrator (170).

11. The respiration type evaluation apparatus (1) according to any one of claims 3 to 10, further comprising a respiration period decider adapted for deciding a respiration period in which an inhalation and an exhalation are performed on the basis of a cycle of variation of the first bioelectrical impedance ($Z_b$) determined by the bioelectrical impedance determiner (170, 200).

12. The respiration type evaluation apparatus (1) according to claim 10, wherein the integrator (170) is adapted for integrating the impedance difference ($\Delta Z$) over a threshold to produce a first integral ($S_1$) of the impedance difference ($\Delta Z$) in an inhalation for each respiration period, and integrates the impedance difference ($\Delta Z$) beneath the threshold to produce a second integral ($S_2$) of the impedance difference ($\Delta Z$) in an exhalation for each respiration period, wherein the respiration type determiner (170) including an integral difference calculator (170) for calculating an integral difference ($\Delta S$) between the first integral ($S_1$) and the second integral ($S_2$) for each respiration period, and wherein the respiration type determiner (170) is adapted for determining whether respiration of the human subject is abdominal or costal on the basis of the integral difference ($\Delta S$) calculated by the integral difference calculator (170).

13. The respiration type evaluation apparatus (1) according to any one of claims 1 to 12, wherein the respiration type determiner (170) is adapted for determining a degree of abdominal respiration and a degree of costal respiration with reference to the respiration of the human subject, the respiration type evaluation apparatus (1) further comprising a first reporter (160) for reporting the degrees determined by the respiration type determiner (170).

14. The respiration type evaluation apparatus (1) according to any one of claims 1 to 13, further comprising a second reporter (160) for instructing the human subject of a time and a depth of inhalation that the human subject should perform and a time and a depth of exhalation that the human subject should perform.

## Fig. 1

Fig. 2

Fig. 3

Fig. 4

Fig. 5

```
              ┌─────────────┐
              │    START     │
              └─────────────┘
                     │
                     ▼
        ┌──────────────────────┐
  S1 ───┤  ENTER PERSONAL      │
        │    INFORMATION       │
        └──────────────────────┘
                     │
                     ▼
        ┌──────────────────────┐
  S2 ───┤   MEASURE            │
        │   THE WEIGHT         │
        └──────────────────────┘
                     │
                     ▼◄──────────────────┐
        ┌──────────────────────┐         │
  S3 ───┤   DETERMINE          │         │
        │   IMPEDANCES         │         │
        └──────────────────────┘         │
                     │                   │
                     ▼                   │
        ┌──────────────────────┐         │
  S4 ───┤│ RESPIRATION        ││         │
        ││ ANALYSIS PROCESS   ││         │
        └──────────────────────┘         │
                     │                   │
                     ▼                   │
        ┌──────────────────────┐         │
  S5 ───┤ SHOW THE RESULTS AND │         │
        │ ADVISORY INFORMATION │         │
        └──────────────────────┘         │
                     │                   │
                     └───────────────────┘
```

Fig. 6

CHEST SKELETAL MUSCLE

ABDOMINAL SKELETAL MUSCLE

LUNG

DIAPHRAGM

VISCERAL TISSUE

Fig. 7A

$Z_a$: BIOELECTRICAL IMPEDANCE AT THE MIDDLE BODY TRUNK

$Z_1$ CHEST SKELETAL MUSCLE

$Z_2$ LUNGS

$Z_3$ ABDOMINAL SKELETAL MUSCLE

$Z_4$ VISCERAL TISSUE

Fig. 7B

$Z_b$: BIOELECTRICAL IMPEDANCE AT THE UPPER BODY TRUNK

$Z_5$ UPPER EXTREMITY SKELETAL MUSCLE

$Z_1$ CHEST SKELETAL MUSCLE

$Z_2$ LUNGS

Fig. 8

$Z_1$ AND $Z_2$ INCREASE DUE TO INHALATION

$Z_2$ DECREACES DUE TO EXHALATION

INHALATION IN COSTAL RESPIRATION

NEUTRAL STATUS

EXHALATION IN ABDOMINAL RESPIRATION

$Z_2$ LUNGS

$Z_1$ CHEST SKELETAL MUSCLE

$Z_2$ LUNGS

$Z_1$ CHEST SKELE-TAL MUSCLE

$Z_2$ LUNGS

$Z_1$ CHEST SKELE-TAL MUSCLE

DIAPHRAGM

DIAPHRAGM

DIAPHRAGM

$Z_4$ VISCERAL TISSUE

$Z_3$ ABDO-MINAL SKELE-TAL MUSCLE

$Z_4$ VISCERAL TISSUE

$Z_3$ ABDO-MINAL SKELE-TAL MUSCLE

$Z_4$ VIS-CER-AL TISSUE

$Z_3$ ABDO-MINAL SKELE-TAL MUSCLE

$Z_3$ AND $Z_4$ DOES NOT CHANGE DURING INHALATION

$Z_3$ AND $Z_4$ INCREASE DUE TO EXHALATION

## Fig. 9

```
            ┌─────────────────────────────────────────┐
            │      RESPIRATION ANALYSIS PROCESS        │
            └─────────────────────────────────────────┘
                              │
                              ▼
        ┌─────────────────────────────────────────────────┐
        │   ACQUIRE THE INSTANTANEOUS VALUES OF THE        │
        │   BIOELECTRICAL IMPEDANCE Zₐ AT THE MIDDLE       │
  S11    │   BODY TRUNK AND OF THE BIOELECTRICAL            │
        │   IMPEDANCE Z_b AT THE UPPER BODY TRUNK          │
        └─────────────────────────────────────────────────┘
                              │
                              ▼
        ┌─────────────────────────────────────────────────┐
        │   CALCULATE THE DIFFERENCE ΔZ BETWEEN THE        │
        │   BIOELECTRICAL IMPEDANCE Zₐ AT THE MIDDLE       │
  S12    │   BODY TRUNK AND THE BIOELECTRICAL IMPEDANCE    │
        │   Z_b AT THE UPPER BODY TRUNK                    │
        └─────────────────────────────────────────────────┘
                              │
                              ▼
        ┌─────────────────────────────────────────────────┐
        │   COMPARE THE DIFFERENCE ΔZ WITH A               │
  S13    │   PREDETERMINED THRESHOLD                        │
        └─────────────────────────────────────────────────┘
                              │
                              ▼
        ┌─────────────────────────────────────────────────┐
        │   DETERMINE WHETHER RESPIRATION OF THE HUMAN     │
  S14    │   SUBJECT IS ABDOMINAL OR COSTAL ON THE BASIS   │
        │   OF COMPARISON                                  │
        └─────────────────────────────────────────────────┘
                              │
                              ▼
                     ┌─────────────────┐
                     │     RETURN      │
                     └─────────────────┘
```

Block S11: ACQUIRE THE INSTANTANEOUS VALUES OF THE BIOELECTRICAL IMPEDANCE $Z_a$ AT THE MIDDLE BODY TRUNK AND OF THE BIOELECTRICAL IMPEDANCE $Z_b$ AT THE UPPER BODY TRUNK

Block S12: CALCULATE THE DIFFERENCE $\Delta Z$ BETWEEN THE BIOELECTRICAL IMPEDANCE $Z_a$ AT THE MIDDLE BODY TRUNK AND THE BIOELECTRICAL IMPEDANCE $Z_b$ AT THE UPPER BODY TRUNK

Block S13: COMPARE THE DIFFERENCE $\Delta Z$ WITH A PREDETERMINED THRESHOLD

Block S14: DETERMINE WHETHER RESPIRATION OF THE HUMAN SUBJECT IS ABDOMINAL OR COSTAL ON THE BASIS OF COMPARISON

Fig. 10

Fig. 11

## Fig. 12

## Fig. 13

RESPIRATION TYPE DETERMINATION PROCESS

S21 — RISING EDGE OF THE COMPARISON RESULT SIGNAL DET1? — NO

YES

S22 — START COUNTING TIME

S23 — TRAILING EDGE OF THE COMPARISON RESULT SIGNAL DET1? — YES

NO

S24 — HAS THE RESPIRATION PERIOD PASSED? — NO

YES

S26 — DETERMINE THAT RESPIRATION IS ABDOMINAL

S25 — DETERMINE THAT RESPIRATION IS COSTAL

## Fig. 14 — RESPIRATION ANALYSIS PROCESS

S31 — ACQUIRE THE INSTANTANEOUS VALUES OF THE BIOELECTRICAL IMPEDANCE $Z_a$ AT THE MIDDLE BODY TRUNK AND OF THE BIOELECTRICAL IMPEDANCE $Z_b$ AT THE UPPER BODY TRUNK

S32 — CALCULATE THE DIFFERENCE $\Delta Z$ BETWEEN THE BIOELECTRICAL IMPEDANCE $Z_a$ AT THE MIDDLE BODY TRUNK AND THE BIOELECTRICAL IMPEDANCE $Z_b$ AT THE UPPER BODY TRUNK

S33 — ON THE BASIS OF THE VARIATION OF THE BIOELECTRICAL IMPEDANCE $Z_b$ AT THE UPPER BODY TRUNK, DETERMINE INDIVIDUAL RESPIRATION PERIODS EACH OF WHICH IS COMPOSED OF AN EXHALATION PERIOD AND AN INHALATION PERIOD

S34 — CALCULATE AN AREA $S_1$ AT WHICH THE DIFFERENCE $\Delta Z$ IS GREATER THAN A THRESHOLD AND AN AREA $S_2$ AT WHICH THE DIFFERENCE $\Delta Z$ IS LESS THAN THE THRESHOLD FOR EACH RESPIRATION PERIOD

S35 — CALCULATE THE DIFFERENCE $\Delta S$ BETWEEN THE AREAS $S_1$ AND $S_2$ FOR EACH RESPIRATION PERIOD

S36 — DETERMINE WHETHER RESPIRATION OF THE HUMAN SUBJECT IS ABDOMINAL OR COSTAL ON THE BASIS OF THE DIFFERENCE $\Delta S$

RETURN

Fig. 15

Fig. 16

Fig. 17

DEPTH OF RESPIRATION

COSTAL

0

ABDOMINAL

TIME

Fig. 18

INHALATION

0

EXHALATION

TIME

Fig. 19

Fig. 20

**EP 2 215 966 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007050127 A